# EUROPEAN PATENT SPECIFICATION

(11) **EP 2 967 930 B1**
(45) Date of publication and mention of the grant of the patent: **28.11.2018**
(21) Application number: 14712175.0
(22) Date of filing: 10.03.2014
(51) Int. Cl.: A61F 2/848, A61F 2/915

(54) **ANTI-MIGRATION TISSUE ANCHORING SYSTEM FOR A FULLY COVERED STENT**
GEWEBEVERANKERUNGSSYSTEM MIT MIGRATIONSSCHUTZ FÜR EINEN VOLLSTÄNDIG BESCHICHTETEN STENT
SYSTÈME D'ANCRAGE DE TISSU ANTI-MIGRATION POUR UNE ENDOPROTHÈSE TOTALEMENT RECOUVERTE

(30) Priority: 13.03.2013 US 201361779414 P
(43) Date of publication of application: 20.01.2016
(73) Proprietor: Boston Scientific Scimed, Inc., Maple Grove, MN 55311-1566 (US)
(72) Inventor: SEDDON, Dane T., Boston, Massachusetts 02127 (US); FLEURY, Sean P., Brighton, MA 02135 (US); WOOD, Mark D., Shrewsbury, Massachusetts 01545 (US); DORAN, Burns P., Monticello, Minnesota 55362 (US); ROSS, Daniel, Watertown, Minnesota 55388 (US)
(74) Representative: Vossius & Partner Patentanwälte Rechtsanwälte mbB
(86) International application number: PCT/US2014/022639
(87) International publication number: WO 2014/159237

(56) References cited:
- EP-A2- 0 732 088
- EP-A2- 0 732 089
- US-A1- 2004 117 004
- US-B1- 6 443 972

## Description

### BACKGROUND OF THE INVENTION

Stents may be implanted in a variety of body lumens or vessels such as within the vascular system, urinary tracts, gastrointestinal tracts, fallopian tubes, coronary vessels, secondary vessels, airways, structural heart (valve frame), etc. They may be self-expanding, expanded by an internal radial force, such as when mounted on a balloon, or a combination of self-expanding and balloon expandable (hybrid expandable). Some stents are partially or fully covered. Migration of the stent from its initial site of implantation can be undesirable.

### BRIEF SUMMARY OF THE INVENTION

Insofar as the terms "invention" and/or "embodiment" are used in the following, and/or features are presented as being optional, this should be interpreted in such a way that the only protection sought is that of the invention as claimed. In at least one embodiment, the prosthesis is a stent comprising a plurality of circumferential bands, a plurality of linking members, at least one anchor. In some embodiments, the prosthesis further includes at least one cover.

In at least one embodiment, the prosthesis is a stent comprising at least one anchoring section comprising a first circumferential band, a second circumferential band, and at least one anchor extending between the first and second circumferential bands. In some embodiments, the anchoring section further includes at least one linking member extending between the first and second circumferential bands. In at least one embodiment, the prosthesis further includes at least one cover.

These and other embodiments which characterize the invention are pointed out with particularity in the claims annexed hereto and forming a part hereof. However, for further understanding of the invention reference can be made to the drawings which form a further part hereof and the accompanying descriptive matter, in which there is illustrated and described embodiments of the invention.

EP 0732088 A2 shows a stent with anchors.

### BRIEF DESCRIPTION OF THE SEVERAL VIEWS OF THE DRAWING(S)

A detailed description of the invention is hereafter described with specific reference being made to the drawings.
FIGS. 1A-D are views of a portion of a prosthesis in an as cut state.
FIGS. 2A-D are views of the twisting of the anchors from the as-cut state of FIGS. 1A-D to the set state.
FIGS. 3A-D are views of the prostheses in FIGS. 2A-D with a cover.
FIG. 4 shows a prosthesis in a deployed state and positioned in a body lumen.

### DETAILED DESCRIPTION OF THE INVENTION

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

In at least one embodiment, the prosthesis is a stent 10. In some embodiments, the stent 10 comprises a plurality of circumferential bands 20, a plurality of linking members 26, at least one anchor 28, and combinations thereof. In at least one embodiment, the prosthesis is a covered stent 10.

As used in this application a "circumferential band", "circumferential ring", "strut column", "serpentine ring", or "serpentine band" are terms identifying the same structure of the stent, specifically a structure formed by a plurality of struts 22 interconnected by a plurality of turns 24 where each turn 24 extends between two struts 22 and each strut 22 extends from two turns 24.

As used in this application, a "turn" 24 refers to either a "peak" 24a (a turn that extends towards the first end of the stent), or a "valley" 24b (a turn that extends towards the second end of the stent). In some embodiments, the circumferential band 20 is closed, as shown in the figures. As used in this application "closed" means that the struts and turns form a continuous pathway that extends about the entire circumference of the stent.

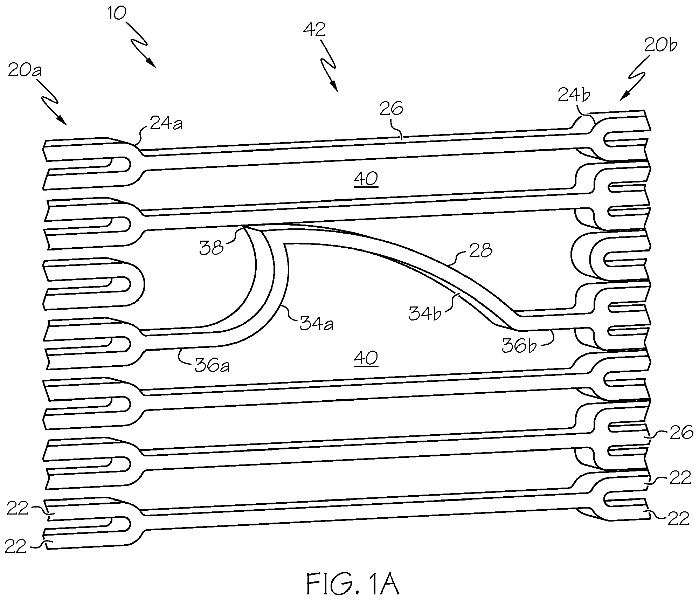

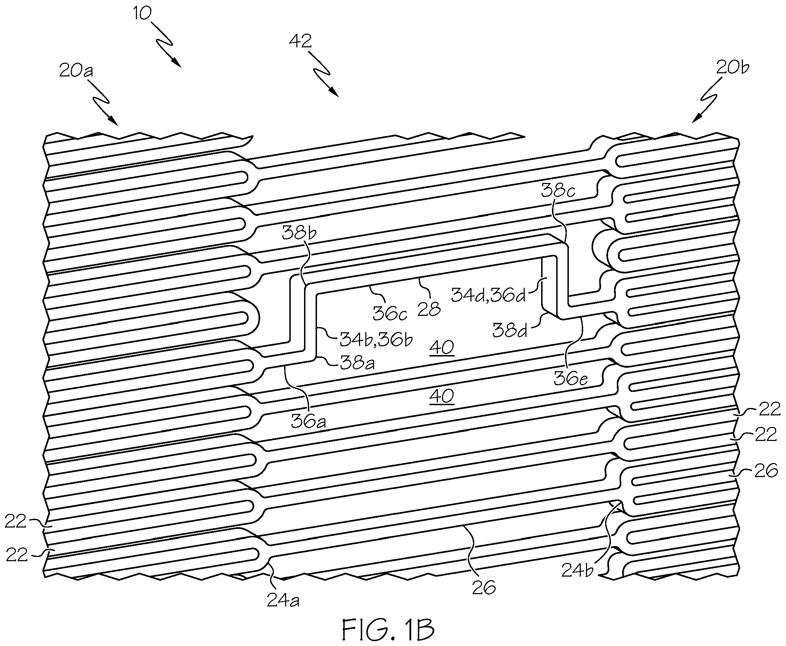

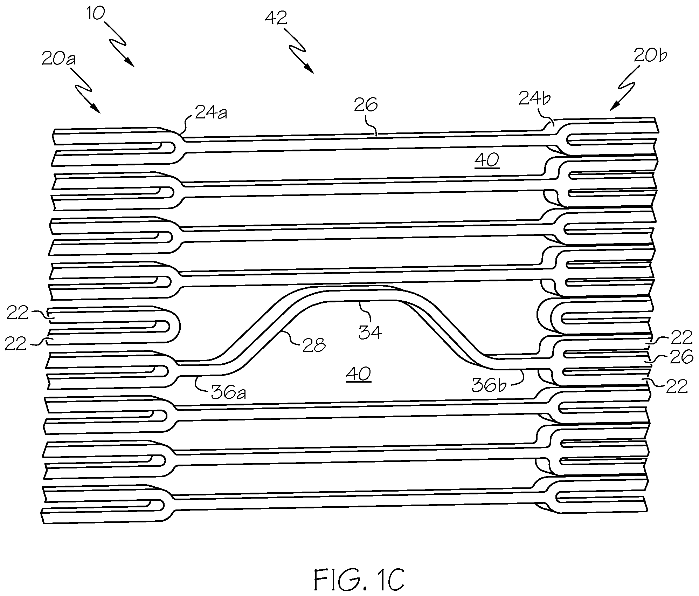

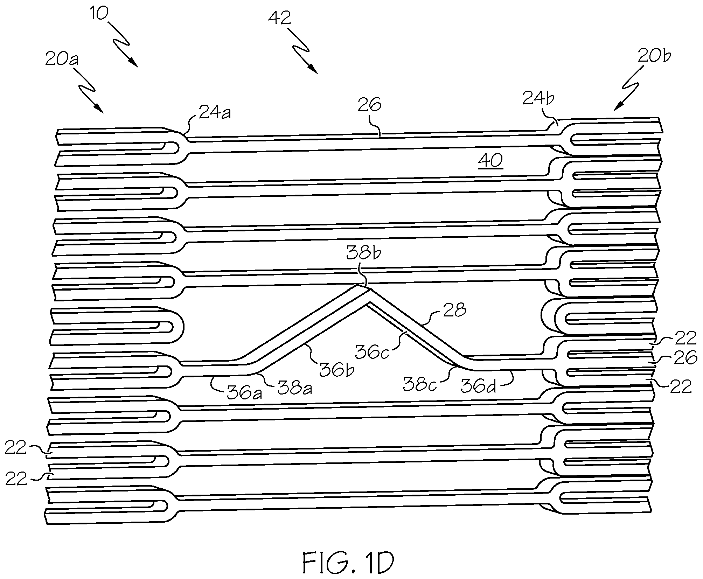

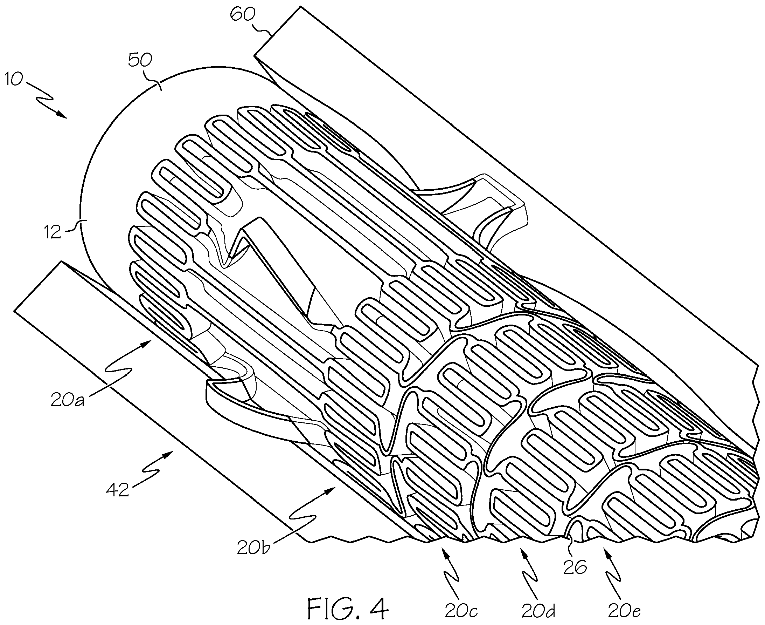

As used in this application, a "linking member" or "connector" 26 connects or engages two circumferential bands 20. A "peak to peak linking member" connects a peak 24a on one circumferential band 20 to a peak 24a on another circumferential band 20. A "peak to valley linking member" 26 connects a peak 24a on one circumferential band 20 to a valley 24b on another circumferential band 20 or vice versa. A "valley to valley linking member" connects a valley 24b on one circumferential band 20 to a valley 24b on another circumferential band 20. It is noted that whether a linking member is a peak to peak linking member or a valley to valley linking member is dependent on the orientation of the stent. Thus, when the stent is oriented in one direction a particular linking member can be a peak to peak linking member, whereas when the stent is oriented in the opposite direction the linking member is a valley to valley linking member. As shown in FIGS. 1-4, the linking members 26 connecting adjacent circumferential bands 20 are peak to valley linking members. As can be seen in FIG. 4, the linking members can be straight or curved.

As used in this application, the terms "connect" or "engage" do not include "indirect" connection or engagement. Thus, for example Element B "connecting" Elements A and C, directly connects A and C with no other element between A and B or between B and C.

The stent and elements forming the stent, such as struts, linking members, and turns, each have a width, length, and thickness. As used in this application, "thickness" is measured radially from the outer surface of the stent to the inner surface of the stent; "width" is measured in a circumferential direction; and "length is measured in a longitudinal direction.

In at least one embodiment, the circumferential bands 20 and linking members 26 form the wall of the stent. In some embodiments, the stent includes a plurality of openings 40 extending through the wall of the stent. As can be seen in FIGS. 1-4, the openings 40 are defined in part by adjacent circumferential bands 20. In some embodiments, the outer surface of the wall is the outer surface of the stent and the inner surface of the wall is the inner surface of the stent. As used in this application, an "inner surface" of the stent is a surface that defines the lumen of the stent and the "outer surface" of the stent is opposite the inner surface.

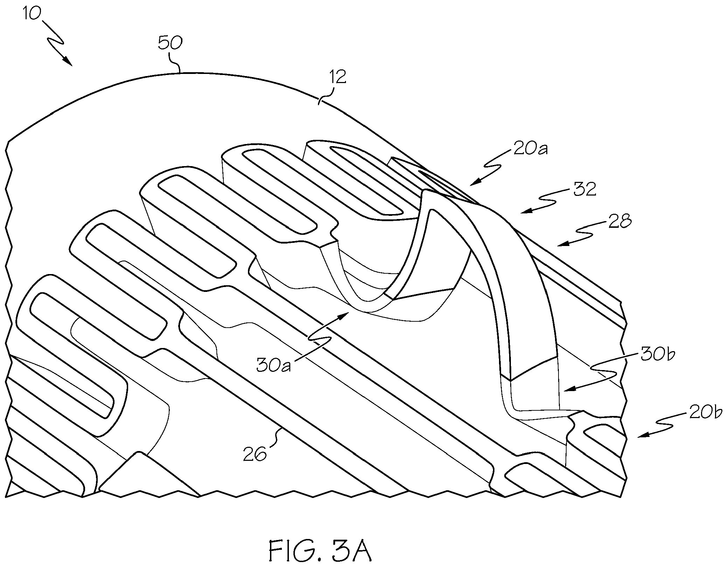

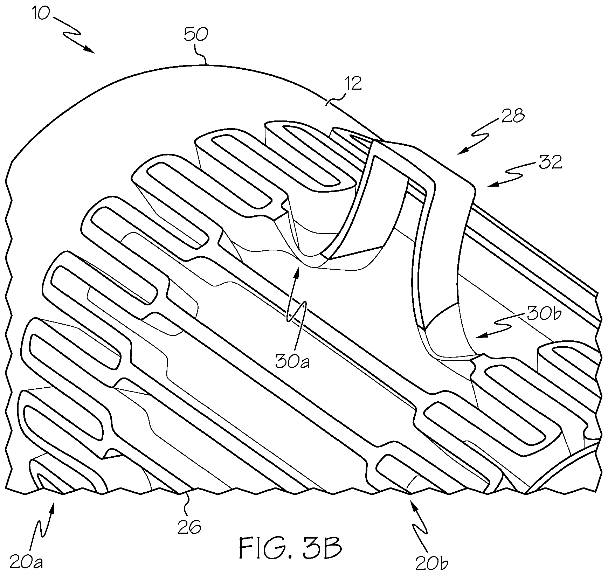

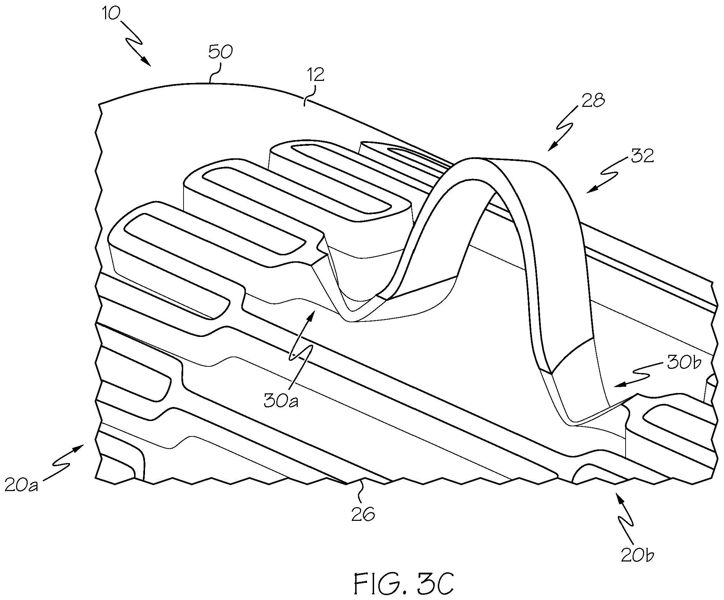

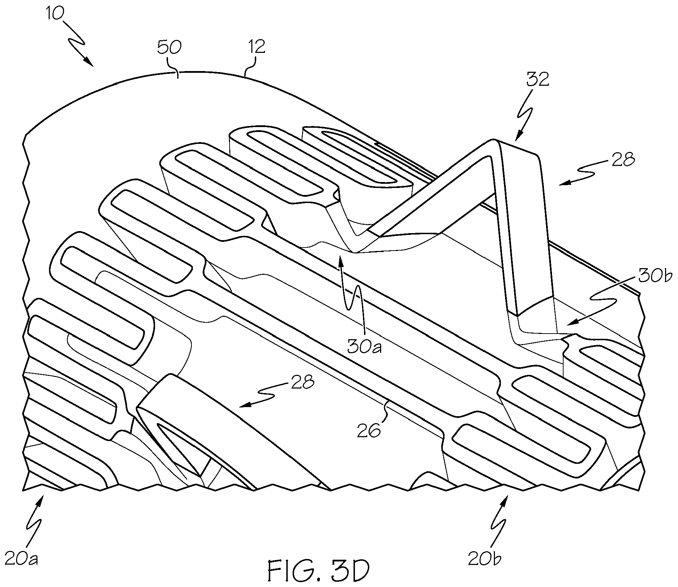

In at least one embodiment, the stent 10 has at least one anchor 28 that is positioned longitudinally adjacent to one or both ends of the stent. In at least one embodiment, the distance of a first end of the anchor 28 from an end of the stent is 0 to 30% of the stent length. In some embodiments, the distance of the anchor 28 from an end of the stent is greater than 0% and no more than 30%. Also as can be seen in the figures, each anchor 28 engages two circumferential bands 20. This is shown for example in FIG. 3-4. Thus, since each end of the anchor is engaged to a circumferential band 20, the anchor 28 has no free ends.

Similar to a linking member 26, an anchor 28 can engage two peaks of adjacent circumferential bands 20 (a peak to peak anchor, not shown); two valleys of adjacent circumferential bands (a valley to valley anchor, not shown); or a peak and a valley of adjacent circumferential bands (a peak to valley anchor, shown for example in FIGS. 1A-D). Thus, in some embodiments, the entire length of the anchor 28 is between two adjacent circumferential bands 20 and in other embodiments, only a portion of the length of the anchor 28 is between two adjacent circumferential bands. In one embodiment, at least the protruding region 32 of the anchor 28 is positioned between two adjacent circumferential bands 20. In at least one embodiment, an anchor 28 has a longitudinal length from about 0.15 inches (3.81 mm) to about 0.25 inches (6.35 mm). Since the anchor extends between two circumferential bands, the two circumferential bands are positioned about 0.225 inches (5.715 mm) to about 0.300 inches (7.620 mm) apart and linking members extending between the two circumferential bands have a longitudinal length of 0.225 inches (5.715 mm) to about 0.275 inches (6.985 mm).

In some embodiments, the stent 10 has one or more anchors 28 are positioned adjacent to only one end of the stent. In other embodiments, the stent has one or more anchors 28 positioned adjacent to both ends of the stent. In at least one embodiment, the anchors 28 are regularly spaced about the circumference of the stent 10. In some embodiments, the stent 10 has two, three, four, five, six or more anchors 28 adjacent to an end of the stent.

In at least one embodiment, the two circumferential bands 20a, 20b and at least one anchor 28 form an anchoring section 42 of the stent 10. In some embodiments, the stent 10 has only one anchoring section 42. In one embodiment, the single anchoring section 42 forms an end region of the stent. In other embodiments, the stent 10 has two anchoring sections, each anchoring section forming an end region of the stent. In at least one embodiment, the anchoring section 42 further includes at least one linking member 26 connecting the first and second circumferential bands 20a, 20b. In some embodiments, the first and second circumferential bands 20a, 20b are out of phase and the linking members 26 are peak to valley linking members. In at least one embodiment, a longitudinal distance between the first and second circumferential bands of the anchoring section is greater than a longitudinal distance between other longitudinally adjacent bands of the stent, as shown for example in FIG. 4. In some embodiments, there are more linking members 26 between the first and second circumferential bands of the anchoring section than between other longitudinally adjacent bands, as shown for example in FIG. 4.

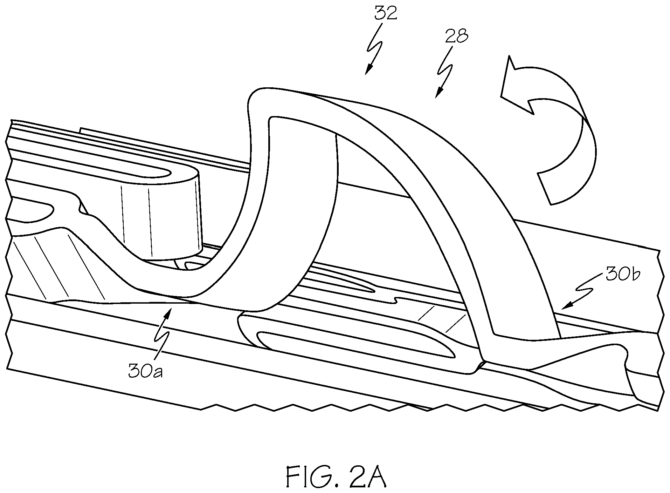

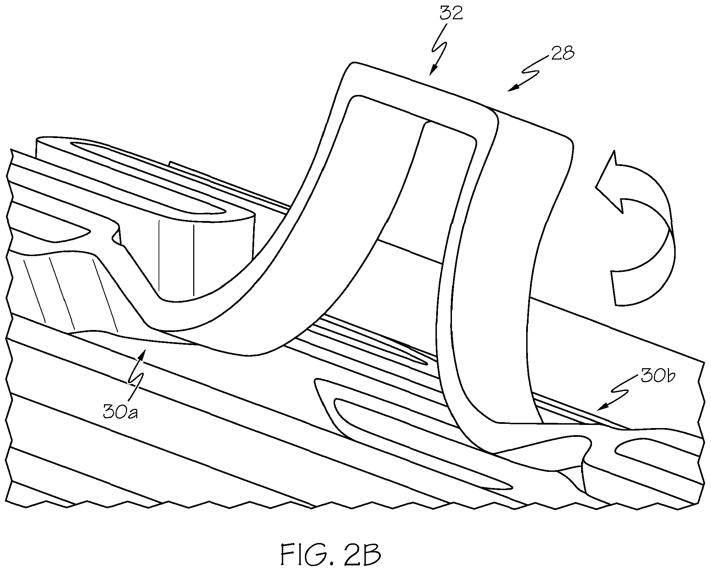

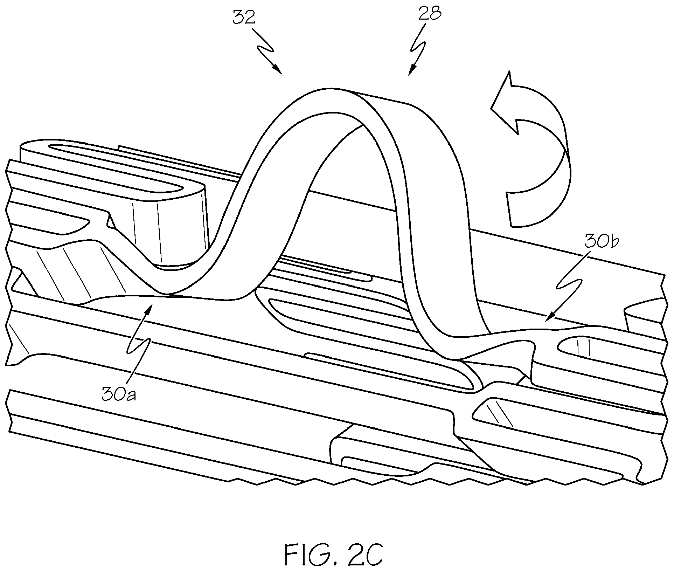

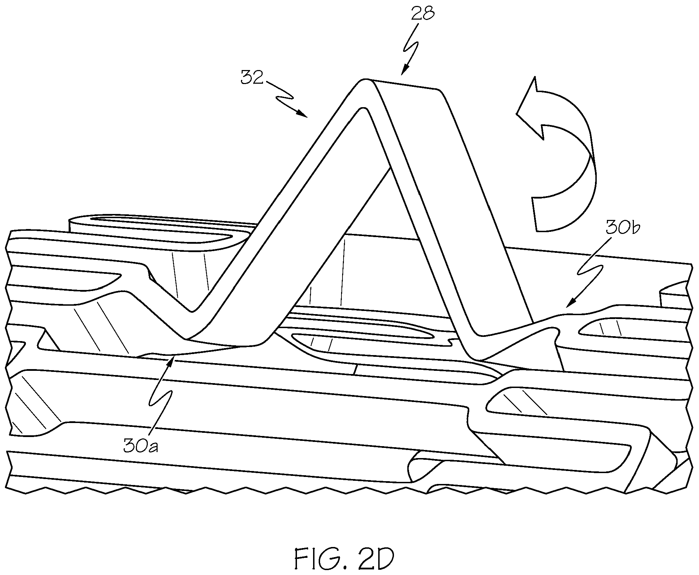

In at least one embodiment, an anchor 28 has an as-cut state and a set state. The anchors 28 in FIGS. 1A-D are in an as-cut state while FIGS. 2A-D show the anchors of FIGS. 1A-D in a set state. As can be seen in FIGS. 1A-D, when the anchor 28 is in the as-cut state, the entire length of the anchor 28 forms a part of the wall of the stent 10 whereas when the anchor 28 is in the set state, a portion of the anchor 28 protrudes from the outer surface of the prosthesis, as can be seen in FIGS. 2A-D. If the stent has no cover, a portion of the anchor in the set state protrudes from the outer surface of the stent. As used herein "protrude" means to extend beyond, or to be positioned above, the outer surface of the prosthesis or the stent. In some embodiments, the anchor 28 protrudes from the cover 50. In at least one embodiment, the anchor 28 is configured to provide for tissue growth around the anchor 28.

In at least one embodiment, the configuration of the anchors 28 is nonlinear. In at least one embodiment, each anchor 28 has at least one bend. The bend can be rounded or sharp. The anchor 28 can have any configuration so long as the anchor in the set state protrudes from the outer surface of the prosthesis. Non-limiting examples of suitable configurations include a wave design, a square design, a rounded design, or a triangular design, as shown for example in FIGS. 1A-D and discussed below in greater detail. The anchors of a stent can have the same design/configuration or different designs/configurations.

In at least one embodiment, the anchor 28 in the set state has a first twist region 30a, a second twist region 30b, and a middle region 32 extending between the two twist regions. In some embodiments, the first twist region 30a, the second twist region 30b, and the middle region 32 of the anchor 28 are positioned between two adjacent circumferential bands 20. In at least one embodiment, peak to peak or valley to valley anchors will further include a straight region that extends from one twist region to the peak or the valley and that is between the two struts engaged to the peak or valley (not shown). In at least one embodiment a twist region 30 of the anchor 28 is a straight region 36 when the anchor 28 is in the as-cut state. In some embodiments, when the anchor is in the set state a first surface of a twist region transitions from being a side surface to an outer surface of the anchor 28 and a second surface opposite the first surface transitions from being a side surface to an inner surface of the anchor 28. Formation of a twist region 30 is discussed in greater detail below.

In some embodiments, each twist region 30 is an end region of the anchor 28. In one embodiment, each twist region 30 is engaged to a circumferential band 20. As can be seen in FIGS. 2A-D, the two twist regions 30 of the anchor provides for a middle region 32 that protrudes from the outer surface of the prosthesis. Thus, the middle region 32 of the anchor 28 can be considered a protruding region or an anchor section.

As can be seen in FIGS. 3A-D and 4, the inner surface of the protruding region 32 of the anchor 28 in the set state is positioned a distance away from the outer surface of the prosthesis and defines an open area of space for tissue growth therearound and therethrough. As used herein an "anchor gap" is the area of space between the anchor and the outer surface of the stent or prosthesis for tissue ingrowth after implantation. In some embodiments, the anchor 28 in the set state has a height of about 0.50 in (1.27 mm) to about 0.10 in (2.54 mm) from the outer surface of the prosthesis or stent. Thus, in one embodiment, middle region 32 of the anchor 28 protrudes a distance of about 0.50 in (1.27 mm) to about 0.10 in (2.54 mm) from the outer surface of the prosthesis or stent.

In some embodiments, an anchor 28 positioned a distance away from an end of the stent mitigates granulation tissue formation at or near the ends of the stent when the stent is implanted in a body lumen. In at least one embodiment, the anchor(s) 28 limit migration of the stent. In this embodiment, the anchor 28 is an anti-migration feature. In at least one embodiment, a fully covered stent with at least one anchor implanted in a body lumen has a reduced stent migration because of tissue growth around the anchor and a reduced luminal occlusion because of the covering. Without being bound by theory, tissue ingrowth limits migration of the stent by anchoring the stent to the luminal wall. In at least one embodiment, the amount of tissue ingrowth around the anchor affects migration. In some embodiments, the longitudinal length of the protruding region 32 of the anchor 28 affects the amount of tissue ingrowth. In at least one embodiment, an anchor with a sharp bend provides for greater tissue excitation and tissue ingrowth than an anchor with a rounded bend. Without being bound by theory, a bend with an edge that is sharper to the touch than another edge that is less sharp will create greater tissue/cell excitation as it rubs against the airway wall thereby inducing inflammation and resulting in tissue ingrowth sooner compared to a bend that has a less sharp edge. Without being bound by theory, minimizing migration of the stent minimizes formation of granulation tissue.

A wave design for an anchor 28 is shown for example in FIG. 1A (as-cut state) and FIG. 3A (set state). In at least one embodiment, the wave design includes a first region that is straight 36a, a first region that is curved 34a, a bend 38a, a second region that is curved 34b, and a second region that is straight 36b. In one embodiment, the wave design further includes a bend connecting the second curved region 34b and the second straight region 36b. In some embodiments, the bend 38a is sharp. In other embodiments, the bend 38a is rounded. As used herein a sharp bend has a smaller radius of curvature than a rounded bend. FIG. 1A shows a bend 38a that is sharp. In at least one embodiment, the first curved region 34a, the bend 38a, and the second curved region 34b form the protruding region 32 of the anchor 28 in the set state. In some embodiments, the anchor gap is defined in part by the first curved region 34a, the bend 38a, and the second curved region 34b of the anchor 28 in the set state. In embodiments with a cover 50 discussed below, the anchor gap is further defined by the outer surface of the cover 50. In at least one embodiment, a wave design provides for aggressive tissue excitation when the stent is implanted in a body lumen. In some embodiments, tissue ingrowth is correlated to tissue excitation. In other embodiments, a wave design provides for an anchor that achieves anchoring of the stent in a body lumen quickly.

A square design for an anchor 28 is shown for example in FIG. 1B (as-cut state) and FIG. 3B (set state). In at least one embodiment, a square design includes a first region that is straight 36a, a first bend 38a, a second region that is either straight 34b or curved 36b, a second bend 38b, a third region that is straight 36c, a third bend 38c, a fourth region that is either straight 34d or curved 36d, a fourth bend 38d, and a fifth region that is straight 36e. In some embodiments, the second and third bends 38b and 38c are rounded. In other embodiments, the second and third bends 38a, 38b are sharp. FIG. 1B is showing rounded second and third bends 38b, 38c. In at least one embodiment, the second region 34b/36b, the first bend 38a, the third region 36c, the second bend 38b, and the fourth region 34d/36d form the protruding region 32 of the anchor 28 in the set state. In some embodiments, the anchor gap is defined in part by the second region 34b/36b, the first bend 38a, the third region 36c, the second bend 38b, and the fourth region 34d/36d of the anchor 28 in the set state. In embodiments with a cover 50 discussed below, the anchor gap is further defined by the outer surface of the cover 50. In some embodiments, the second and fourth regions are straight 36 when the anchor is in the as-cut state and curved 34 when the anchor is in the set state (compare FIGS. 1B and 3B). In other embodiments, the second and fourth regions are straight 36 when the anchor 28 is in the as-cut state and when the anchor 28 is in the set state (not shown). In some embodiments, the third region 36c is substantially parallel to the outer surface of the prosthesis. In at least one embodiment, a square design provides for a large volume of tissue ingrowth around the anchor when the stent is implanted in a body lumen. Without being bound by theory, the longitudinal length of the protruding region of the anchor affects the volume of tissue ingrowth.

A rounded design for an anchor 28 is shown for example in FIG. 1C (as-cut state) and FIG. 3C (set state). In at least one embodiment, the rounded design includes a first region that is straight 36a, a second region that is curved 34, and a third region that is straight 36b. In some embodiments, the second region 34 includes two straight regions and a curved region connecting the two straight regions. In at least one embodiment, the curved region 34 forms the protruding region 32 of the anchor 28 in the set state. In some embodiments, the anchor gap is defined in part by the curved region 34 of the anchor 28 in the set state. In embodiments with a cover 50 discussed below, the anchor gap is further defined by the outer surface of the cover 50. In some embodiments, a rounded design provides for very little tissue excitation when the stent is implanted in a body lumen. In at least one embodiment, a stent with anchors having a rounded design is implanted at a location where the tissue is inflamed prior to implantation of the stent.

A triangular design for an anchor 28 is shown for example in FIG. 1D (as-cut state) and FIG. 3D (set state). In at least one embodiment, the triangular design includes a first region that is straight 36a, a first bend 38a, a second region that is straight 36b, a second bend 38b, a third region that is straight 36c, a third bend 38c, and a fourth region that is straight 36d. In some embodiments, the second bend 38b is rounded. In other embodiments, the second bend 38b is sharp. In at least one embodiment, the second straight region 36b, the second bend 38b, and the third straight region 36c form the protruding region 32 of the anchor 28 in the set state. In some embodiments, the anchor gap is defined in part by the second straight region 36b, the second bend 38b, and the third straight region 36c of the anchor 28 in the set state. In embodiments with a cover 50 discussed below, the anchor gap is further defined by the outer surface of the cover 50. In some embodiments, a triangular design causes mild tissue excitation when the stent is implanted in a body lumen. In other embodiments, a triangular design provides for relatively quick anchoring of a stent after implantation in a body lumen.

The stent 10 as has several states, an "as cut state," followed by a "heat set state," followed by a "crimped state," followed by a "deployed state." As used in this application, a stent is in an "as cut state" after laser cutting and prior to heat setting; a stent is in the "heat set state" after the as cut state and after being heat treated; a stent is in a "crimped state" when positioned on a delivery device; and a stent is in the "deployed state" when it is deployed in a body lumen. FIG. 4 shows the stent 10 deployed in a vessel 60. As shown in FIG. 4, the anchors 28 are embedded in the wall of the vessel 60.

In at least one embodiment, the stent 10 has a cover 50, as shown for example in FIGS. 3A-D. In some embodiments, the stent 10 has a cover 50 when the stent 10 is in the crimped state and when the stent 10 is in the deployed state. As used herein, a "cover" 50 extends over the openings 40 defined by the stent wall thereby occluding the openings and preventing tissue growth through the openings and into the lumen of the prosthesis. In at least one embodiment, the prosthesis 10 is a fully covered stent. As used herein a "fully covered stent" has a cover 50 that extends at least from the first end to the second end of the stent. Thus, the cover 50 of a fully covered stent has a length equal to or greater than the length of the stent 10. As used herein a "covered stent" has a cover 50 with a longitudinal length less than the longitudinal length of the stent. The cover 50 can be positioned over a) the outer surface of the wall of the stent; b) the inner surface of the wall of the stent; or c) both the inner surface and outer surface of the wall of the stent. In some embodiments, the cover 50 has a thickness that is a) less than the thickness of the wall of the stent; b) equal to the thickness of the wall of the stent; or c) greater than the thickness of the wall of the stent. Reference hereinafter to the outer surface 12 of the prosthesis includes a) embodiments where the outer surface 12 of the prosthesis is formed by the stent and the cover 50, and b) embodiments where the outer surface 12 of the prosthesis is formed only by the cover 50.

In some embodiments the stent, the delivery system or other portion of the assembly may include one or more areas, bands, coatings, members, etc. that is (are) detectable by imaging modalities such as X-Ray, MRI, ultrasound, etc. In some embodiments at least a portion of the stent and/or adjacent assembly is at least partially radiopaque.

In some embodiments the at least a portion of the stent 10 is configured to include one or more mechanisms for the delivery of a therapeutic agent. Often the agent will be in the form of a coating or other layer (or layers) of material placed on a surface region of the stent, which is adapted to be released at the site of the stent's implantation or areas adjacent thereto. A layer or coating of therapeutic agent as used herein is not a cover 50 as used herein because the therapeutic agent does not extend over and occlude the openings defined by the wall of the stent. In some embodiments, the protruding region 32 of the anchor 28 has a therapeutic agent deposited thereon.

As used herein a "therapeutic agent" is a drug or other pharmaceutical product used to treating, preventing, or alleviating the symptoms of disease. Therapeutic agents include non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent is delivered by a polymer agent, the polymer agent may be a polystyrene-polyisobutylene-polystyrene triblock copolymer (SIBS), polyethylene oxide, silicone rubber and/or any other suitable substrate.

In at least one embodiment, the stent 10, in each of the several states, comprises an outer surface 12, an inner surface 14, a longitudinal length extending from a first end to a second end, a plurality of circumferential bands 20, a plurality of linking members 26, and at least one anchor 28. In some embodiments, the at least one anchor 28 is in the as-cut state when the stent is in the as-cut state. In other embodiments, the at least one anchor 28 is in the set state when the stent is in the crimped state and when the stent is in the deployed state. Thus, the delivery device is configured to carry a stent with at least one anchor 28 in the set state.

In at least one embodiment, a method of forming a stent includes forming the as-cut state of the stent 10; transforming the at least one anchor 28 from the as-cut state to the set state; and optionally applying a cover 50 to the stent. As an additional option, a therapeutic agent is selectively applied to the protruding portions of the anchors 28.

These steps are discussed below in greater detail.

### Step of Forming the as-cut state of the stent:

In some embodiments, the as-cut state of the stent 10 is formed by laser cutting or etching a pattern in a tube of stent material. In other embodiments, the as-cut state of the stent 10 is formed by laser cutting or etching a pattern in a flat sheet of stent material, and forming a tube by rolling and joining the long edge of the sheet.

In some embodiments, the pattern includes a plurality of circumferential bands 20, a plurality of linking members 26, and at least one anchor 28, as discussed above. In other embodiments, the pattern includes an anchoring section 42. In some embodiments, the anchor 28 in the as-cut state has a thickness of about 0.01 inches (0.25 mm).

Any other suitable technique which is known in the art or which is subsequently developed may also be used to form a stent disclosed herein.

The stent 10 may be made from any suitable biocompatible materials including one or more polymers, one or more metals or combinations of polymer(s) and metal(s). Examples of suitable materials include biodegradable materials that are also biocompatible. By biodegradable is meant that a material will undergo breakdown or decomposition into harmless compounds as part of a normal biological process. Suitable biodegradable materials include polylactic acid, polyglycolic acid (PGA), collagen or other connective proteins or natural materials, polycaprolactone, hylauric acid, adhesive proteins, co-polymers of these materials as well as composites and combinations thereof and combinations of other biodegradable polymers. Other polymers that may be used include polyester and polycarbonate copolymers. Examples of suitable metals include, but are not limited to, stainless steel, titanium, tantalum, platinum, tungsten, gold and alloys of any of the above-mentioned metals. Examples of suitable alloys include platinum-iridium alloys, cobalt-chromium alloys including Elgiloy and Phynox, MP35N alloy and nickel-titanium alloys, for example, Nitinol.

The inventive stents may be made of shape memory materials such as superelastic Nitinol or spring steel, or may be made of materials which are plastically deformable. In the case of shape memory materials, the stent may be provided with a memorized shape and then deformed to a reduced diameter shape for delivery to a body lumen. The stent may restore itself to its memorized shape in a body lumen upon being heated to a transition temperature and having any restraints removed therefrom.

### Step of Transforming the at least one anchor from the as-cut state to the set state:

In at least one embodiment, the anchor 28 in the as-cut state is transformed to an anchor in a set state. In some embodiments, transforming the anchor 28 from the as-cut state to the set state comprises twisting or turning the anchor 28 outward so that a portion of the anchor protrudes from the outer surface of the prosthesis or stent. In at least one embodiment, when the anchor 28 is in the set state, a surface of a twist region 30 of the anchor 28 transitions from being a side surface to being an outer surface of the anchor 28. This can be seen for example in FIGS. 2A-D. As can be seen, the side surface is adjacent to an end of the anchor and the outer surface is adjacent to the middle region of the anchor. In at least one embodiment, the width of the anchor 28 in the set state is equal to the thickness of the anchor 28 in the as-cut state. In at least one embodiment, transforming the anchor 28 from the as-cut state to the set state comprises forming one or more twist regions 30. In some embodiments, one or more twist regions are formed by twisting or turning the anchor 28 outward so that a portion of the anchor protrudes from the outer surface of the prosthesis or stent.

In at least one embodiment, the anchor 28 is heat set after being twisted to maintain the set state of the anchor.

As discussed above, the wall of the stent 10 has a thickness. In some embodiments, when the anchor 28 is in the cut state, the anchor has a thickness equal to the thickness of the wall, and when the anchor is in the set state, the anchor has a width equal to the thickness of the wall of the stent. This can be seen for example in FIGS. 1-2.

### Step of Applying a cover to the stent:

In at least one embodiment, a cover 50 is applied to the stent 10. In some embodiments, a cover 50 is applied to the stent 10 after the one or more anchors 28 have been heat set to the set state. In some embodiments, the cover 50 has a length at least equal to the length of the stent 10. In other embodiments, the cover 50 has a length greater than the length of the stent 10.

In a least one embodiment, the stent is a fully covered stent. A cover 50 can be applied in any suitable manner that covers and obstructs the openings 40 in the wall of the stent 10. In some embodiments, the cover 50 is applied by dip coating the stent 10. In other embodiments, the cover 50 is applied by spray-coating coating material onto the stent 10.

In at least one embodiment, cover material applied to is removed to form the anchor gap. In some embodiments, cover material applied to the surface of the protruding region 32 of the anchor 28 are also removed so that the protruding region 32 is exposed from, or bare of, the cover 50. In other words, the protruding region 32 has no cover material thereon. Any method to remove the cover material to form the anchor gap or to remove the cover material on the surfaces of the anchor 28 can be used so long as the cover 50 is not punctured. As discussed above, the cover 50 prevents the lumen of the prosthesis from being occluded by tissue ingrowth. In some embodiments, a laser is used to form the anchor gap and to remove cover material on the protruding region 32 of the anchor 28. A suitable laser is a YAG laser. In some embodiments, the stent is held in place while the cover material is being removed. In one embodiment, the stent is on a mandrel. In other embodiments, the anchor gap is filled with an epoxy during the coating process and after the coating has been applied, the epoxy is dissolved to form the anchor gap. In one laser is used to remove cover material from the anchor so that the protruding region 32 of the anchor 28 is bare. In still other embodiments, coating in the anchor gap is punched out with a dye or other device with a shape that matches the geometry of the anchor gap. In one laser is used to remove cover material from the anchor so that the protruding region 32 of the anchor 28 is bare.

Suitable materials for the cover 50 include any other type of material that prevents tumor or tissue ingrowth through at least some of the openings 40. Non-limiting examples include silicone elastomers, polyurethane, polystyrene-polyisobutylene-polystyrene triblock copolymer (SIBS), ePTFE, and combinations thereof.

The following numbered statements are directed to one or more aspects of a stent or prosthesis as described above:
Statement 1. A stent comprising:
   a first circumferential band;
   a second circumferential band;
   at least one anchor connected to both of the first and second circumferential bands, wherein each anchor defines an anchor gap.
Statement 2. The stent of statement 1, each anchor comprising:
   a first anchor end region connected to the first circumferential band;
   a second anchor end region connected to the second circumferential band; and
   a middle region defining the anchor gap.
Statement 3. The stent of statement 1-2, the first anchor end region and the second anchor end region each being a twist region.
Statement 4. The stent of statements 1-3, each anchor having a wave design, a square design, a rounded design, or a triangular design.
Statement 5. The stent of statements 1-4, each anchor being a peak to peak anchor, a peak to valley anchor, or a valley to valley anchor.
Statement 6. The stent of statements 1-5, the at least one anchor being a plurality of anchors.
Statement 7. The stent of statements 1-6, the first circumferential band, the second circumferential band, and the at least one anchor forming an anchor section.
Statement 8. The stent of statement 7, the anchor section further comprising a plurality of linking members connecting the first and second circumferential bands.
Statement 9. The stent of statement 8, each linking member being straight.
Statement 10. The stent of statements 7-9, the stent having only one anchor section.
Statement 11. The stent of statements 1-10, further comprising a cover to form a covered prosthesis.
Statement 12. The stent of statement 11, wherein the middle region protrudes from the cover for a distance.
Statement 13. The stent of statements 11-12, wherein the middle region has no cover material thereon.
Statement 14. The stent of statements 11-13, wherein the cover comprises silicone, polyurethane, and combinations thereof.
Statement 15. A stent having an expanded state and an unexpanded state, the stent comprising:
   an anti-migration section comprising:
      a first circumferential band;
      a second circumferential band;
      at least one anti-migration feature extending between the first and second circumferential bands;
   wherein a portion of the anti-migration feature protrudes from an outer surface of the stent when the stent is in the unexpanded state and when the stent is in the expanded state.
Statement 16. The stent of statement 15, each anti-migration feature further defining a gap between an inner surface of each anti-migration feature and the outer surface of the stent.
Statement 17. The stent of statements 15-16, each anti-migration feature being a peak to peak anti-migration feature, a peak to valley anti-migration feature, or a valley to valley anti-migration feature.
Statement 18. The stent of statements 15-17, each anti-migration feature comprising a first end region and a second end region, the first end region being a twist region and the second end region being a twist region.
Statement 19. The stent of statements 15-18, further comprising a cover, wherein the portion of the anti-migration feature protruding from the outer surface of the stent is exposed.
Statement 20. The stent of statement 19, each anchor gap being further defined by the cover.
Statement 21. A method of making an intraluminal prosthesis comprising:
   laser cutting a stent, the stent comprising a first circumferential band, a second circumferential band and at least one anchor from a tube, each anchor connected to the first circumferential band and to the second circumferential band, the stent having an outer surface; and
   twisting each anchor to protrude a portion of the anchor outward from the outer surface of the stent.
Statement 22. The covered prosthesis of statement 21, each anchor being a peak to peak anchor, a peak to valley anchor, or a valley to valley anchor.
Statement 23. The method of statement 21-22, each anchor comprising a first anchor end region connected to the first circumferential band and a second anchor end region connected to the second circumferential band, wherein both the first and second end regions are twisted to protrude the portion of the anchor from the outer surface of the stent.
Statement 24. The method of statements 21-23, wherein after the anchor is twisted, the anchor defines an anchor gap.
Statement 25. The method of statements 21-24, further comprising heat setting each anchor after twisting the anchor.
Statement 26. The method of statement 24-25, the method further comprising filling the anchor gap with epoxy.
Statement 27. The method of statements 21-26, further comprising forming a cover by applying a cover material to the stent.
Statement 28. The method of statement 26, wherein the cover material comprises silicone, polyurethane, and combinations thereof.
Statement 29. The method of statements 27-28, further comprising removing any cover material from the anchor gap.
Statement 30. The method of statement 29, further comprising removing the cover material from each anchor so that the portion of the anchor protruding from the cover is exposed.
Statement 31. The method of statements 29-30, wherein a laser is used to form the anchor gap, to ablate the anchor, or to form the anchor gap and to ablate the anchor.
Statement 32. The method of statement 29, wherein cover material is removed from the anchor gap by punching a die sized for the anchor gap.
Statement 33. The method of statement 31, further comprising removing the cover material from each anchor so that the portion of the anchor protruding from the cover is exposed.
Statement 34. The method of statements 27-28, further comprising removing the epoxy from the anchor gap after forming the cover.

The above disclosure is intended to be illustrative and not exhaustive. This description will suggest many variations and alternatives to one of ordinary skill in this art. The various elements shown in the individual figures and described above may be combined or modified for combination as desired. All these alternatives and variations are intended to be included within the scope of the claims where the term "comprising" means "including, but not limited to".

Further, the particular features presented in the dependent claims can be combined with each other in other manners within the scope of the invention such that the invention should be recognized as also specifically directed to other embodiments having any other possible combination of the features of the dependent claims. For instance, for purposes of claim publication, any dependent claim which follows should be taken as alternatively written in a multiple dependent form from all prior claims which possess all antecedents referenced in such dependent claim if such multiple dependent format is an accepted format within the jurisdiction (e.g. each claim depending directly from claim 1 should be alternatively taken as depending from all previous claims). In jurisdictions where multiple dependent claim formats are restricted, the following dependent claims should each be also taken as alternatively written in each singly dependent claim format which creates a dependency from a prior antecedent-possessing claim other than the specific claim listed in such dependent claim below.

This completes the description of the invention. Those skilled in the art may recognize other equivalents to the specific embodiment described herein which equivalents are intended to be encompassed by the claims attached hereto.

## Claims

1. A stent (10) comprising:
a first circumferential band;
a second circumferential band;
at least one anchor (28) connected to both of the first and second circumferential bands, wherein each anchor (28) defines an anchor gap; and
a cover (50), wherein the at least one anchor (28) protrudes from the cover (50).

2. The stent (10) of claim 1, each anchor (28) comprising:
a first anchor end region (30a) connected to the first circumferential band;
a second anchor end region (30b) connected to the second circumferential band; and
a middle region (32) defining the anchor gap, wherein the middle region (32) protrudes from the cover (50) for a distance.

3. The stent (10) of claim 2, the first anchor end region (30a) and the second anchor end region (30b) each being a twist region.

4. The stent (10) of claim 1, each anchor (28) having a wave design, a square design, a rounded design, or a triangular design.

5. The stent (10) of claim 1, each anchor (28) being a peak to peak anchor, a peak to valley anchor, or a valley to valley anchor.

6. The stent (10) of claim 1, the first circumferential band, the second circumferential band, and the at least one anchor forming an anchor section, wherein the anchor section comprises a plurality of linking members (26) connecting the first and second circumferential bands.

7. The stent (10) of claim 6, each linking member (26) being straight.

8. The stent (10) of claim 2, wherein the middle region (32) has no cover material thereon.

9. The stent (10) of claim 1, wherein the cover (50) comprises silicone, polyurethane, and combinations thereof.

10. The stent (10) of claim 1 having an expanded state and an unexpanded state,
wherein a portion of the at least one anchor (28) forms an anti-migration feature, the portion protrudes from an outer surface of the stent (10) when the stent (10) is in the unexpanded state and when the stent (10) is in the expanded state.

11. A method of making a stent according to claim 1, comprising:
laser cutting a stent (10), the stent (10) comprising a first circumferential band, a second circumferential band and at least one anchor (28) from a tube, each anchor connected to the first circumferential band and to the second circumferential band, the stent (10) having an outer surface;
twisting each anchor (28) to protrude a portion of the anchor (28) outward from the outer surface of the stent (10) to define an anchor gap; and
forming a cover (50) by applying a cover material to the stent (10) such that at least one anchor (28) protrudes from the cover (50).

12. The method of claim 11, each anchor (28) comprising a first anchor end region (30a) connected to the first circumferential band and a second anchor end region (30b) connected to the second circumferential band, wherein both the first and second end regions are twisted to protrude the portion of the anchor (28) from the outer surface of the stent (10).

13. The method of claim 11, further comprising heat setting each anchor (28) after twisting the anchor (28).

14. The method of claim 11, the method further comprising removing any cover material from the anchor gap after forming the cover (50).

## Patentansprüche

1. Stent (10), der aufweist:
ein erstes Umfangsband;
ein zweites Umfangsband;
mindestens einen Anker (28), der sowohl mit dem ersten als auch dem zweiten Umfangsband verbunden ist, wobei jeder Anker (28) eine Ankerlücke definiert; und
eine Hülle (50), wobei der mindestens eine Anker (28) aus der Hülle (50) vorsteht.

2. Stent (10) nach Anspruch 1, wobei jeder Anker (28) aufweist:
einen ersten Ankerendbereich (30a), der mit dem ersten Umfangsband verbunden ist;
einen zweiten Ankerendbereich (30b), der mit dem zweiten Umfangsband verbunden ist; und
ein mittleren Bereich (32), der die Ankerlücke definiert, wobei der mittlere Bereich (32) um eine Strecke aus der Hülle (50) vorsteht.

3. Stent (10) nach Anspruch 2, wobei der erste Ankerendbereich (30a) und der zweite Ankerendbereich (30b) jeweils ein Windungsbereich sind.

4. Stent (10) nach Anspruch 1, wobei jeder Anker (28) eine Wellengestaltung, eine quadratische Gestaltung, eine abgerundete Gestaltung oder eine dreieckige Gestaltung aufweist.

5. Stent (10) nach Anspruch 1, wobei jeder Anker (28) ein Spitze-zu-Spitze-Anker, ein Spitze-zu-Tal-Anker oder ein Tal-zu-Tal-Anker ist.

6. Stent (10) nach Anspruch 1, wobei das erste Umfangsband, das zweite Umfangsband und der mindestens eine Anker einen Ankerabschnitt bilden, wobei der Ankerabschnitt mehrere Verbindungselemente (26) aufweist, die das erste und zweite Umfangsband verbinden.

7. Stent (10) nach Anspruch 6, wobei jedes Verbindungselement (26) gerade ist.

8. Stent (10) nach Anspruch 2, wobei der mittlere Bereich (32) kein Hüllmaterial darauf aufweist.

9. Stent (10) nach Anspruch 1, wobei die Hülle (50) Silikon, Polyurethan und Kombinationen davon aufweist.

10. Stent (10) nach Anspruch 1, der einen ausgedehnten Zustand und einen nicht ausgedehnten Zustand aufweist,
wobei ein Abschnitt des mindestens einen Ankers (28) ein Antimigrationsmerkmal bildet, und der Abschnitt aus einer Außenfläche des Stents (10) vorsteht, wenn sich der Stent (10) im nicht ausgedehnten Zustand befindet und wenn sich der Stent (10) im ausgedehnten Zustand befindet.

11. Verfahren zum Herstellen eines Stents nach Anspruch 1, das aufweist:
Laserschneiden eines Stents (10), wobei der Stent (10) ein erstes Umfangsband, ein zweites Umfangsband und mindestens einen Anker (28) aufweist, aus einer Röhre, wobei jeder Anker mit dem ersten Umfangsband und dem zweiten Umfangsband verbunden ist, wobei der Stent (10) eine Außenfläche aufweist;
Verwinden jedes Ankers (28), so dass ein Abschnitt des Ankers (28) aus der Außenfläche des Stents (10) nach außen vorsteht, um eine Ankerlücke zu definieren; und
Bilden einer Hülle (50) durch Aufbringen eines Hüllmaterials auf den Stent (10) so dass mindestens ein Anker (28) aus der Hülle (50) vorsteht.

12. Verfahren nach Anspruch 11, wobei jeder Anker (28) einen ersten Ankerendbereich (30a), der mit dem ersten Umfangsband verbunden ist, und einen zweiten Ankerendbereich (30b) aufweist, der mit dem zweiten Umfangsband verbunden ist, wobei sowohl der erste als auch der zweite Endbereich verwunden sind, so dass der Abschnitt des Ankers (28) aus der Außenfläche des Stents (10) vorsteht.

13. Verfahren nach Anspruch 11, das ferner das Thermofixieren jedes Ankers (28) nach dem Verwinden des Ankers (28) aufweist.

14. Verfahren nach Anspruch 11, wobei das Verfahren ferner das Entfernen irgendeines Hüllmaterials von der Ankerlücke nach dem Bilden der Hülle (50) aufweist.

## Revendications

1. Endoprothèse (10), comprenant :
une première bande circonférentielle ;
une deuxième bande circonférentielle ;
au moins un ancrage (28) raccordé à la première ainsi qu'à la deuxième bande circonférentielle, chaque ancrage (28) définissant un interstice d'ancrage ; et
une couverture (50), ledit au moins un ancrage (28) étant en saillie sur la couverture (50).

2. Endoprothèse (10) selon la revendication 1, chaque ancrage (28) comprenant :
une première zone d'extrémité d'ancrage (30a) raccordée à la première bande circonférentielle ;
une deuxième zone d'extrémité d'ancrage (30b) raccordée à la deuxième bande circonférentielle ; et
une zone centrale (32) définissant l'interstice d'ancrage, ladite zone centrale (32) étant en saillie à distance de la couverture (50).

3. Endoprothèse (10) selon la revendication 2, où la première zone d'extrémité d'ancrage (30a) et la deuxième zone d'extrémité d'ancrage (30b) sont chacune une zone de torsion.

4. Endoprothèse (10) selon la revendication 1, où chaque ancrage (28) a une forme ondulée, une forme carrée, une forme arrondie ou forme triangulaire.

5. Endoprothèse (10) selon la revendication 1, où chaque ancrage (28) est un ancrage de sommet à sommet, un ancrage de sommet à fond ou un ancrage de fond à fond.

6. Endoprothèse (10) selon la revendication 1, où la première bande circonférentielle, la deuxième bande circonférentielle et ledit au moins un ancrage forment une section d'ancrage, ladite section d'ancrage comprenant une pluralité d'éléments de liaison (26) raccordant la première et la deuxième bandes circonférentielles.

7. Endoprothèse (10) selon la revendication 6, où chaque élément de liaison (26) est rectiligne.

8. Endoprothèse (10) selon la revendication 2, où la zone centrale (32) n'est pas recouverte par un matériau de couverture.

9. Endoprothèse (10) selon la revendication 1, où la couverture (50) comprend de la silicone, du polyuréthane, et des combinaisons de ceux-ci.

10. Endoprothèse (10) selon la revendication 1, présentant un état d'expansion et un état de non-expansion,
où une partie dudit au moins un ancrage (28) forme un élément anti-migration, la partie fait saillie d'une surface extérieure de l'endoprothèse (10) quand l'endoprothèse (10) est en état de non-expansion et quand l'endoprothèse (10) est en état d'expansion.

11. Procédé de fabrication d'une endoprothèse selon la revendication 1, comprenant :
la découpe par laser d'une endoprothèse (10) dans un tube, ladite endoprothèse (10) comprenant une première bande circonférentielle, une deuxième bande circonférentielle et au moins un ancrage (28), chaque ancrage étant raccordé à la première ainsi qu'à la deuxième bande circonférentielle, ladite endoprothèse (10) présentant une surface extérieure ;
la torsion de chaque ancrage (28) pour faire saillir une partie de l'ancrage (28) vers l'extérieur depuis la surface extérieure de l'endoprothèse (10) afin de définir un interstice d'ancrage ; et
la formation d'une couverture (50) par application d'un matériau de couverture sur l'endoprothèse (10) de telle manière qu'au moins un ancrage (28) fait saillie sur la couverture (50).

12. Procédé selon la revendication 11, où chaque ancrage (28) comprend une première zone d'extrémité d'ancrage (30a) raccordée à la première bande circonférentielle et une deuxième zone d'extrémité d'ancrage (30b) raccordée à la deuxième bande circonférentielle, où la première ainsi que la deuxième zones d'extrémité sont tordues pour faire saillir la partie de l'ancrage (28) sur la surface extérieure de l'endoprothèse (10).

13. Procédé selon la revendication 11, comprenant en outre la thermofixation de chaque ancrage (28) après torsion de l'ancrage (28).

14. Procédé selon la revendication 11, où ledit procédé comprend en outre le retrait de tout matériau de couverture de l'interstice d'ancrage après formation de la couverture (50).
